(19) Europäisches Patentamt
European Patent Office
Office européen des brevets

(11) **EP 3 967 220 B1**

(12) **EUROPÄISCHE PATENTSCHRIFT**

(45) Veröffentlichungstag und Bekanntmachung des Hinweises auf die Patenterteilung:
**23.08.2023 Patentblatt 2023/34**

(21) Anmeldenummer: **21195954.9**

(22) Anmeldetag: **10.09.2021**

(51) Internationale Patentklassifikation (IPC):
*A61B 5/0536* (2021.01)    *A61B 5/08* (2006.01)
*A61B 5/091* (2006.01)    *A61B 5/00* (2006.01)

(52) Gemeinsame Patentklassifikation (CPC):
**A61B 5/0809; A61B 5/0536; A61B 5/091;**
**A61B 5/742;** A61B 5/7253

(54) **VERARBEITEN UND VISUALISIEREN VON DATEN EINER ELEKTRISCHEN IMPEDANZTOMOGRAPHIE**

PROCESSING AND VISUALIZING DATA OF ELECTRICAL IMPEDANCE TOMOGRAPHY

TRAITER ET VISUALISER DES DONNÉES D'UNE TOMOGRAPHIE D'IMPÉDANCE ÉLECTRIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.09.2020 DE 102020211471**

(43) Veröffentlichungstag der Anmeldung:
**16.03.2022 Patentblatt 2022/11**

(73) Patentinhaber: **Universität Leipzig**
**04109 Leipzig (DE)**

(72) Erfinder:
• **Schneider, Dominic**
**04109 Leipzig (DE)**
• **Neumuth, Thomas**
**04109 Leipzig (DE)**
• **Salz, Peter**
**04109 Leipzig (DE)**
• **Reske, Andreas**
**04105 Leipzig (DE)**

(74) Vertreter: **Pfenning, Meinig & Partner mbB**
**Patent- und Rechtsanwälte**
**An der Frauenkirche 20**
**01067 Dresden (DE)**

(56) Entgegenhaltungen:
DE-A1-102017 007 224    DE-B3-102015 006 902

• **ZHAO ZHANQI ET AL: "Comparison of different functional EIT approaches to quantify tidal ventilation distribution", PHYSIOLOGICAL MEASUREMENT, Bd. 39, Nr. 1, 30. Januar 2018 (2018-01-30), Seite 01NT01, XP055886308, DOI: 10.1088/1361-6579/aa9eb4**

**Beschreibung**

Die vorliegende Erfindung betrifft eine Vorrichtung und ein Computerprogrammprodukt zum Verarbeiten und Visualisieren von Daten einer elektrischen Impedanztomographie (EIT).

Vorrichtungen zum Durchführen einer elektrischen Impedanztomographie oder Elektro-Impedanz-Tomographie sind aus dem Stand der Technik bereits bekannt. Hierbei werden mittels Elektro-Impedanz-Messungen Signale aufgenommen und aus daraus gewonnenen Daten und Datenströme ein oder mehrere Bilder generiert. Diese Bilder zeigen Unterschiede in der elektrischen Leitfähigkeit verschiedener Körpergewebe wie Knochen oder Haut bzw. bei Organen, insbesondere der Lunge. Beispielsweise offenbart die Druckschrift WO 01/93760 A1 eine derartige Vorrichtung und ein entsprechendes Verfahren. Aus der Publikation Girrbach F, Landeck T, Schneider D, et al. Detection of post-traumatic pneumothorax using electrical impedance tomography-An observer blinded study in pigs with blunt chest trauma, PLoS One. 2020;15(1):e0227518 ist bekannt, dass durch Untersuchungen an gesunden und kranken Schweinen auf eine Schädigung der Lunge geschlossen werden kann. Aus dem Patent DE 10 2015 006 902 B3 ist eine Vorrichtung zur Verarbeitung und Visualisierung von EIT-Daten bekannt, bei der eine Visualisierung von regionalen Ventilations-Verzögerungen der Lunge möglich ist.

DE 10 2016 107 603 A1 offenbart ein Benutzerinterface eines medizinischen Diagnosesystems, dem in Echtzeit EIT-Daten zugeführt werden. Eine zeitreihenbasierte Auswertung von EIT-Daten ist in der Druckschrift EP 2 762 062 A1 gezeigt. Das Dokument DE 601 24 541 T2 behandelt ein Verfahren und eine Vorrichtung zur Darstellung von Information, die durch elektrische Impedanz-Tomografie erhalten ist.

Insbesondere bei Funktionsstörungen der Lunge ist eine zuverlässige und schnelle Erkennung von Belüftungsstörungen nötig, die mit einer konventionellen Elektro-Impedanz-Tomographie allerdings nur unzureichend gewährleistet werden kann. Beispielsweise ist bei einer Pneumothoraxdetektion ein schnelles und zuverlässiges Ergebnis der Messmethode gewünscht und nötig, was aber bislang nicht mit der gewünschten Genauigkeit und Geschwindigkeit in einer schnell erfassbaren Darstellung erfolgen kann.

Der vorliegenden Erfindung liegt daher die Aufgabe zugrunde, eine Vorrichtung vorzuschlagen, die die genannten Nachteile vermeidet, mit der also eine schnelle und präzise Messung samt einfach wahrnehmbarer Darstellung ermöglicht wird.

Diese Aufgabe wird erfindungsgemäß gelöst durch eine Vorrichtung nach Anspruch 1. Vorteilhafte Ausgestaltungen und Weiterbildungen sind in den abhängigen Ansprüchen beschrieben.

Bei einem zum Verständnis der Erfindung nützlichen, aber von der Erfindung nicht umfassten Verfahren zum Verarbeiten und Visualisieren von Daten einer elektrischen Impedanztomographie einer Lunge werden zunächst Daten mittels eines Elektro-Impedanz-Tomographie-Geräts aufgenommen und eine visuelle Darstellung der Daten generiert. Anschließend wird mindestens ein Tidalbild eines Atemzyklus gebildet und jeder Pixel des mindestens einen Tidalbilds anhand einer vorab definierten Funktion transformiert. Durch das Transformieren wird jedem Pixel des mindestens einen Tidalbilds ein Absolutwert, vorzugsweise einen Farbwert, zugeordnet, der einer Wahrscheinlichkeit für ein Auftreten eines bestimmten Ereignisses entspricht.

Durch die Transformation kann somit anhand der vorab definierten Funktion, d. h. einer Funktion, die samt Parametern bereits vor dem Durchführen des Verfahrens bekannt ist, ein in einfacher Art und Weise visuell wahrnehmbarer Indikator für das vorab bestimmte Ereignis, insbesondere eine Belüftungsstörung einer Lunge oder ein anderes physiologisches Signal, angegeben werden, der durch seine Position im Tidalbild auch räumlich definiert ist. Das Verfahren kann jedoch auch lediglich für eine Überwachung einer Atemfunktion, d. h. insbesondere Einatmen und Ausatmen, verwendet werden, wobei dann die Atemfunktion das bestimmte Ereignis darstellt. Das Verfahren ist schnell durchführbar, da die Funktion der Transformation bereits vorab festgelegt worden ist, liefert aber dennoch mit ausreichend hoher Genauigkeit die verlangten Informationen. Zudem kann das Verfahren auch mit bereits existierenden Geräten durchgeführt werden, da typischerweise lediglich eine Nachrüstung der Software erfolgt. Unter den Daten der elektrischen Impedanztomographie bzw. Elektro-Impedanz-Tomographie sollen hierbei Signale oder Daten verstanden werden, die mit einer Vorrichtung zum Durchführen der elektrischen Impedanztomographie, die auch als Elektro-Impedanz-Tomographie-Gerät bezeichnet wird, gewonnen werden. Hierzu werden in der Regel mittels einer Gruppe von Elektroden bzw. eines Elektrodengürtels Messsignale in verschiedener Ausprägung erfasst, beispielsweise eine elektrische Spannung und ein elektrischer Strom bzw. daraus abgeleitete elektrische Widerstandswerte oder Impedanzwerte. Die zunächst erzeugte visuelle Darstellung der Daten kann, muss aber nicht zwangsläufig ausgegeben werden.

Unter dem Begriff "Tidalbild" soll im Rahmen dieser Schrift ein Bild verstanden werden, das zu einem bestimmten Zeitpunkt eines Atemzyklus örtlich bzw. räumlich aufgelöst eine Verteilung lokaler Impedanzen in einer transversalen Ansicht oder in einer aus mehreren transversalen Ansichten zusammengesetzten dreidimensionalen Ansicht wiedergibt. Das Tidalbild bezieht sich dabei in der Regel auf die Lunge, muss aber nicht die gesamte Lunge umfassen, sondern kann auch nur auf einzelne Regionen, sogenannte "regions of interest" gerichtet sein.

Typischerweise werden die Impedanzen während eines Messzyklus fortlaufend ermittelt. Als Messzyklus oder Frame soll hierbei eine zeitlich nacheinander erfolgende Einspeisung an mindestens eine Elektrode oder ein Elektrodenpaar mit jeweils zugehörigem Messumlauf an einer oder mehreren weiteren Elektroden verstanden werden. Das Verfahren ist prinzipiell auf ein einziges Tidalbild anwendbar, typischerweise werden jedoch mehrere Tidalbilder untersucht.

Für das Transformieren des mindestens einen Tidalbilds anhand der vorab definierten Funktion wird eine nicht-lineare Sigmoid-Funktion verwendet.

Entgegen der naheliegenden Lösung einer linearen Transformation erfolgt bei Verwenden einer Sigmoid-Funktion überraschenderweise eine einfach und schnell durchzuführende Transformation, die dennoch zuverlässig Informationen über die Belüftungsstörung liefert.

Vorzugsweise werden Parameter der nicht-linearen Funktion bzw. der Sigmoid-Funktion vorab anhand eines Histogramms gewählt bzw. angepasst. Das Histogramm soll hierbei eine Differenz zwischen Daten einer elektrischen Impedanztomographie gesunder Individuen und von Individuen mit Belüftungsstörungen der Lunge aufweisen. Durch diese einfache Kopplung mit bereits bekannten Messwerten erfolgt eine zuverlässige Detektion von Belüftungsstörungen.

Unmittelbar vor dem Transformieren des mindestens einen Tidalbilds kann anhand der vorab definierten Funktion eine Normalisierung durchgeführt werden. Als niedrigster Wert von Absolutwerten bzw. Farbwerten für die Normalisierung wird hierbei jedoch ein Mittelwert von Absolutwerten bzw. Farbwerten von Randpixeln des mindestens einen Tidalbilds verwendet. Als Randpixel sollen hierbei insbesondere diejenigen Pixel verstanden werden, die direkt an den Rand angrenzen oder in einer Entfernung von bis zu fünf Pixeln, typischerweise zwei Pixeln oder drei Pixeln, von diesem Rand positioniert sind. Es können Pixel eines einzelnen Randbereichs herangezogen werden, es ist aber auch möglich, Pixel mehrerer Randbereiche, bei einem rechteckigen Bild also von bis zu vier Randbereichen, heranzuziehen. Als Mittelwert kann sowohl ein arithmetischer Mittelwert, ein geometrischer Mittelwert als auch ein Median verwendet werden. Durch diese Art der Normalisierung können die relevanten Pixel und deren Farbwerte bei der Transformation mit höherem Kontrast bzw. größerer Genauigkeit angegeben werden. Mit dem beschriebenen Verfahren wird zudem eine höhere Robustheit, insbesondere gegenüber falsch positiven Werten, erreicht. Der Mittelwert des Randes entspricht somit in der Regel einem neuen Nullwert. Es kann in diesem Fall auch negative Werte geben, die dann pathologisch sind und einer Belüftungsstörung entsprechen. Liegen hingegen auch nach der Normalisierung keine negativen Werte vor, ist auch die Wahrscheinlichkeit für das Auftreten einer Belüftungsstörung gering.

Vor dem Transformieren des mindestens einen Tidalbilds kann eine Filterung, vorzugsweise eine Tiefpassfilterung oder eine Bandpassfilterung durchgeführt werden, um Störgrößen bzw. Störsignale zu entfernen, insbesondere Perfusionssignale und langsamere Impedanzänderungen mit einer Frequenz kleiner 5 pro Minute.

Es kann vorgesehen sein, das transformierte Tidalbild, d. h. das Tidalbild nach der Transformation auf einer Ausgabeeinheit visuell erkennbar darzustellen, um beispielsweise menschlichen Benutzern eine einfach wahrnehmbare Information zur Position der Belüftungsstörung anzubieten. Vorzugsweise erfolgt die Darstellung in Falschfarbendarstellung, um einem menschlichen Benutzer einen schnell wahrnehmbaren Informationsgewinn zu verschaffen.

Eine Vorrichtung zum Verarbeiten und Visualisieren von Daten einer elektrischen Impedanztomographie einer Lunge weist eine Aufnahmeeinheit auf, die dazu ausgebildet ist, Daten einer elektrischen Impedanztomographie zu erfassen. Alternativ zu der Aufnahmeeinheit kann auch eine Eingabeeinheit vorgesehen sein, die dazu eingerichtet ist, mittels einer elektrischen Impedanztomographie aufgenommene Daten zur Weiterverarbeitung zu erhalten. Die Vorrichtung, die auch als Elektro-Impedanz-Tomographie-Gerät bezeichnet werden kann, weist außerdem eine Auswerteeinheit auf, die dazu ausgebildet ist, eine visuelle Darstellung der Daten zu generieren und mindestens ein Tidalbild eines Atemzyklus zu bilden. Die Auswerteeinheit ist weiter dazu eingerichtet, jeden Pixel des mindestens einen Tidalbilds anhand einer vorab definierten Funktion zu transformieren, wobei die Transformation jedem der Pixel des mindestens einen Tidalbilds einen Absolutwert, insbesondere einen Farbwert, zuordnet, der einer Wahrscheinlichkeit für ein Auftreten eines bestimmten Ereignisses entspricht.

Die Vorrichtung, insbesondere die Auswerteeinheit, kann entsprechend die zuvor beschriebene Sigmoid-Funktion samt deren Parametern in einer Speichereinheit gespeichert aufweisen. Ebenso können Daten des Histogramms in dieser Speichereinheit gespeichert sein und von der Auswerteeinheit verwendet werden. Die Auswerteeinheit ist somit dazu eingerichtet, für das Transformieren des mindestens einen Tidalbilds anhand der vorab definierten Funktion eine nicht-lineare Funktion, erfindungsgemäß eine Sigmoid-Funktion, zu verwenden, wobei besonders vorzugsweise Parameter der Sigmoid-Funktion vorab anhand eines Histogramms gewählt werden, wobei das Histogramm eine Differenz zwischen Daten einer elektrischen Impedanztomographie gesunder Individuen und an dem bestimmten Ereignis erkrankter Individuen aufweist. Die Auswerteeinheit ist schließlich vorzugsweise auch zum Durchführen der Normalisierung und bzw. oder der Filterung ausgebildet. Die Ausgabeeinheit bildet typischerweise einen Teil der beschriebenen Vorrichtung. Es kann vorgesehen sein, dass die Ausgabeeinheit dazu ausgebildet ist, das transformierte Tidalbild visuell erkennbar darzustellen, typischerweise in einer Falschfarbendarstellung.

Das beschriebene nicht erfindungsgemäße Verfahren kann mit der beschriebenen Vorrichtung durchgeführt werden, d. h. die beschriebene Vorrichtung kann zum Durchführen des beschriebenen Verfahrens ausgebildet sein.

Ein Computerprogrammprodukt enthält eine Befehlsfolge, die die beschriebene Vorrichtung zum Verarbeiten und Visualisieren von Daten einer elektrischen Impedanztomographie ansteuert und bzw. oder das beschriebene Verfahren durchführt, wenn die Befehlsfolge in der Vorrichtung durchgeführt bzw. ausgeführt wird. Die genannte Befehlsfolge ist hierzu typischerweise in der Speichereinheit gespeichert.

Ausführungsbeispiele der Erfindung sind in den Zeichnungen dargestellt und werden nachfolgend anhand der Figuren 1 bis 5 erläutert.

Es zeigen:

Fig. 1 eine schematische Ansicht einer Vorrichtung zum Verarbeiten und Visualisieren von Daten einer elektrischen Impedanztomographie;

Fig. 2 ein schematisches Ablaufdiagramm eines entsprechenden Verfahrens;

Fig. 3 Tidalbilder in verschiedenen Verfahrensstadien;

Fig. 4 ein Histogramm mit Differenzen zwischen Daten einer elektrischen Impedanztomographie gesunder Individuen und Individuen mit Belüftungsstörungen und

Fig. 5 ein Diagramm einer verwendete Sigmoid-Funktion.

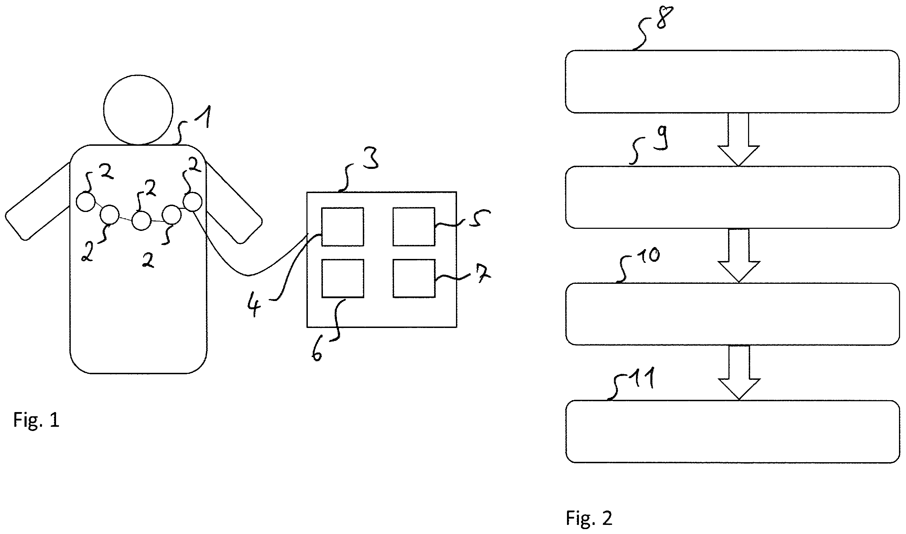

In Figur 1 ist in einer schematischen Ansicht eine Vorrichtung 3 zum Verarbeiten und Visualisieren von Daten einer elektrischen Impedanztomographie gezeigt, die auch als Elektro-Impedanz-Tomographie-Gerät bezeichnet wird. Generell wird hierbei eine Aufnahme des Thorax bzw. Brustkorbs angefertigt, auf der die Lunge als Zielorgan erfasst wird. Das zu überwachende Individuum 1, beispielsweise ein Mensch, wird im Bereich seiner Lunge mit mehreren Elektroden 2 oder Elektrodenpaaren versehen. Die Vorrichtung 3 verfügt über eine Steuereinheit 6, beispielsweise einen Mikroprozessor mit einer Speichereinheit, durch die ein elektrisches Stromsignal bzw. ein elektrisches Spannungssignal über die Elektroden 2 zum Körper des Individuums 1 gelangt. Durch eine Aufnahmeeinheit 4 der Vorrichtung 3 wird nachfolgend die resultierende elektrische Spannung gemessen. Stromeinspeisung und Spannungsmessung erfolgen dabei in der Regel an Elektrodenpaaren, die im Allgemeinen einen fixen Abstand zwischen den jeweiligen Elektroden 2 aufweisen, d. h. typischerweise bilden zwei unmittelbar benachbarte Elektroden 2 ein Paar. Für jedes Einspeiseelektrodenpaar werden beispielsweise alle anderen Elektrodenpaare durchlaufen und die elektrische Spannung gemessen.

Wurden alle Einspeiseelektrodenpaare und alle Messelektrodenpaare durchlaufen, werden die erhaltenen Messwerte als Frame bezeichnet. Aus den erhaltenen Messwerten bzw. Daten kann nun durch die Auswerteeinheit 5 der Vorrichtung 3 durch einen Rekonstruktionsalgorithmus ein zweidimensionales Bild, bei mehreren Elektrodengürteln auch ein dreidimensionales Bild rekonstruiert werden, beispielsweise mittels des GREIT-Algorithmus. Je nach Geschwindigkeit der Messung resultiert ein entsprechend aufgelöster Bilddatenstrom, der sich im Hinblick auf medizinische Fragestellungen analysieren lässt. Das Ergebnis kann schließlich auf einer Ausgabeeinheit 7, beispielsweise einem Display oder einem Monitor, visuell erkennbar für einen menschlichen Benutzer ausgegeben werden. Die an die Ausgabeeinheit 7 zur Darstellung übermittelten Daten liegen typischerweise als Bildsignaldaten oder Videosignaldaten vor. Basierend auf diesem, in der Regel in Falschfarbendarstellung analog einer Orientierung des ursprünglichen Tidalbilds 12 wiedergegebenen Tidalbild 12 kann dann der menschliche Benutzer, beispielsweise ein Arzt, eine Interpretation der dargestellten Daten vornehmen, um daraus auf das Vorliegen von Belüftungsstörungen zu schlussfolgern. Hohe Wahrscheinlichkeiten für einen Bereich mit einer Belüftungsstörung können beispielsweise einen hellen Farbwert aufweisen und damit nicht nur die Position bzw. Lokalisation, sondern auch die Größe und, bei mehreren Livebildern, auch dynamische Veränderungen ausgeben. Anstelle einer Falschfarbendarstellung kann auch eine beliebige andere, für einen Menschen visuell wahrnehmbare Darstellung erfolgen, bei welcher einzelne Pixel oder Bereiche beispielsweise mit einer Farbe, einer Schraffur, einer grafischen Kodierung auf Basis von Grautönen, einer Helligkeit, einer Farbtransparenz, einer Farbsättigung oder einem Muster bildlich dargestellt werden.

In Figur 2 ist schematisch ein Ablaufdiagramm eines Verfahrens zum Verarbeiten und Visualisieren von Daten einer elektrischen Impedanztomographie eines Thorax mit einer Lunge gezeigt. Wiederkehrende Merkmale sind in dieser Figur wie auch in den folgenden Figuren mit identischen Bezugszeichen versehen. In einem ersten Schritt 8, der in der Regel mit der Aufnahmeeinheit 4 durchgeführt wird, erfolgt das nichtinvasive Aufnehmen der Daten mittels der Vorrichtung 3. Alternativ hierzu können die entsprechenden Daten bereits aufgenommen worden sein und werden lediglich bereitgestellt, so dass das gesamte Verfahren losgelöst von dem Individuum 1 und gegebenenfalls auch räumlich getrennt von dem Individuum 1 durchgeführt werden. Aus diesen Daten wird in Schritt 9 eine visuelle Darstellung der Daten generiert, beispielsweise ein Bild, bei dem jedem Pixel ein Farbwert bzw. ein Grauwert als initialer Absolutwert zugeordnet wird. Anschließend wird ein Tidalbild 12 eines Atemzyklus der Lunge gebildet, d. h. eine Folge mehrerer Tidalbilder, die die Lunge an verschiedenen Zeitpunkten während des Atemzyklus zeigen. In Schritt 11 wird jeder Pixel des Tidalbilds 12 anhand einer vorab definierten Funktion transformiert. Bei dem Transformieren bzw. der Transformation wird jedem Pixel des Tidalbilds 12 anhand des Pixelwerts, also des initialen Absolutwerts des Pixels, eine Wahrscheinlichkeit und daraus ein Farbwert als Absolutwert nach der Transformation zugeordnet, der einer Wahrscheinlichkeit für ein Auftreten der Belüftungsstörung entspricht. Das Tidalbild 12 als Differenzbild zwischen maximaler Ausatmung und Einatmung kann hierbei erhalten werden, indem im Bilddatenstrom ein Zeitpunkt mit minimaler Impedanz betrachtet wird und der folgende Zeitpunkt mit maximaler Impedanz gesucht sowie die Werte der beiden Bilder voneinander abgezogen werden. Somit wird mit jedem Atemzug ein neues Tidalbild 12 generiert. Das Verfahren ist auf eine zeitliche Folge von Tidalbildern 12 ausgelegt, funktioniert aber prinzipiell auch nur mit einem einzigen Bild. Die Verfahrensschritte 9, 10, und 11 werden in der Regel von der Auswerteeinheit 5 durchgeführt. Das Tidalbild kann eine Auflösung von 64 Pixeln mal 64 Pixeln haben, es ist aber auch möglich, eine höhere Auflösung oder eine niedrigere Auflösung, zum Beispiel 32 Pixel mal 32 Pixel zu verwenden.

Hierbei kann auch vorgesehen sein, eine Vorverarbeitung, in der Regel ebenfalls durch die Auswerteeinheit 5, vor Schritt 9 durchzuführen, bei der eine Abtrennung eines Atemsignals von einem ermittelten Gesamtsignal erfolgt.

Das extrahierte Atemsignal sollte so wenige Störsignale wie möglich beinhalten, insbesondere keine Durchblutungsanteile. Da die genaue Form des Atemsignals nur eine untergeordnete Rolle spielt, bietet sich vorzugsweise eine Tiefpassfilterung der von der Vorrichtung 3 aufgenommenen Daten an. Es kann aber auch eine Bandpassfilterung oder eine Fourier-Transformation bzw. Wavelet-Transformation oder ein darauf basierendes Verfahren durchgeführt werden. Alternativ oder zusätzlich kann auch Mode-Decomposition oder Non-Negative Matrix Factorization Anwendung finden.

Bei der Tidalbildberechnung in Schritt 10 werden im dargestellten Ausführungsbeispiel von der Auswerteeinheit 5 der minimale Wert und der maximale Wert im jeweiligen Tidalbild 12 bestimmt und jeweils auf Null bzw. 255 gesetzt. Die restlichen Werte im Bild werden auf die entsprechenden, linear interpolierten Zwischenwerte gesetzt. Dieser Schritt kann alternativ auch vor der Filterung erfolgen. Diese Normalisierung der Daten ist vorteilhaft, da absolute Werte der jeweiligen Pixel keine unmittelbare Aussagekraft haben, weil sie auch von Individuum zu Individuum stark unterschiedlich ausgeprägt sein können.

In einer Variante dieses Schritts 10 wird nicht der minimale Wert des gesamten Bilds bzw. aller Pixel bestimmt, sondern ein mittlerer Wert der Randpixel des rekonstruierten Tidalbilds 12 bestimmt. Dieser Wert wird dann auf Null gesetzt und das globale Maximum wie gehabt auf 255, alle anderen Werte werden linear interpoliert. Hierbei können auch negative Werte auftreten, die in der Regel als pathologisch anzusehen sind und eine Belüftungsstörung anzeigen. In dieser Ausgestaltung werden falsch positiv erkannte Belüftungsstörungen minimiert, die auf Bildartefakten basieren.

Bei der Datentransformation in Schritt 11 erfolgt somit eine Zuordnung von Auftretenswahrscheinlichkeiten zu den einzelnen Werten der Bildpunkte, d. h. jedem Bildpunkt wird, anhand seines Wertes im Tidalbild 12, die Wahrscheinlichkeit seines Auftretens in einem EIT-Tidalbild 12 mit Belüftungsstörungen zugewiesen, und ist damit ein Maß für das Vorhandensein einer Belüftungsstörung am entsprechenden Bildpunkt. Dies erfolgt mithilfe einer Funktion, die aus einem Vergleich zwischen gesunden Lungengewebe und Lungengewebe mit einer Belüftungsstörung stammt. Die Funktion wird vor dem Durchführen des in Figur 2 dargestellten Verfahrens automatisiert oder manuell an die Daten angepasst und optimiert. Prinzipiell werden unterschiedliche Transformationsfunktionen erhalten, je nachdem, welche Arten von Trainingsdatensätzen verwendet werden. Bei einer Verwendung von Daten mit singulärem Pneumothorax wird daher eine Transformationsfunktion erhalten, die Wahrscheinlichkeiten für einen Pneumothorax ausgibt. Existieren daneben noch andere Belüftungsstörungen wie zum Beispiel ein Lungenkollaps, werden andere Parameter für die Transformationsfunktion erhalten, so dass diese Art der Belüftungsstörung besser detektierbar ist. Allerdings erlaubt das Verfahren generell keine eindeutige Differenzierung bezüglich der Art der Belüftungsstörung, insbesondere bei mittleren und niedrigen Wahrscheinlichkeiten.

Es wird somit jedem Wert f(x) eines Pixels $\times$ eine Wahrscheinlichkeit W zugeordnet, wobei W aus [0, 1] ist, und W die Wahrscheinlichkeit dafür ist, dass der Pixel $\times$ Teil einer Belüftungsstörung ist bzw. dass eine solche an diesem Pixel anliegt. Nachfolgend wird der Wahrscheinlichkeit W ein Falschfarbenwert für die bildliche Darstellung zugeordnet.

Somit wird gerade nicht die naheliegende Lösung einer linearen Transformation gewählt, sondern es wird eine nicht-lineare Transformation mit einer Sigmoid-Funktion verwendet, die auch als "squashing function" bezeichnet wird. Das in Figur 2 schematisch beschriebene Verfahren wird wie beschrieben mit der Vorrichtung 3 durchgeführt.

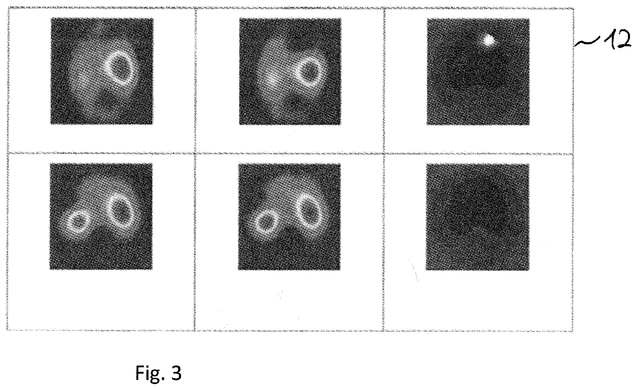

In Figur 3 sind zwei verschiedene Tidalbilder 12 untereinander in der linken Spalte dargestellt. Die mittlere Spalte zeigt die entsprechenden Tidalbilder 12 nach der Filterung, während in der rechten Spalte das Tidalbild 12 nach der Transformation 11 wiedergegeben ist. Bei den Tidalbildern 12 ist in der oberen Reihe das Vorliegen eines Pneumothorax anhand der Farbwerte in dem Tidalbild 12 der rechten Spalte erkennbar, während bei den Bildern der unteren Zeile keine Belüftungsstörung vorliegt.

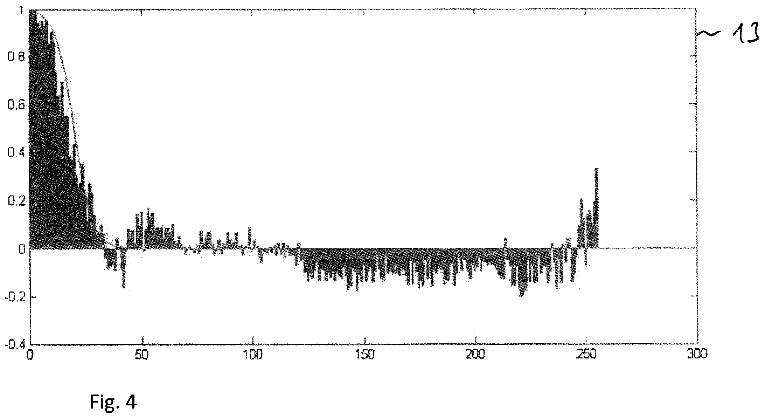

Figur 4 schließlich zeigt ein beispielhaftes Histogramm 13, das mit den zuvor beschriebenen Schritten anhand der Daten von Schweinen erhalten wurde.

Hierbei wurden Paare von Schweinedaten untersucht, wobei die Paare aus einem Datensatz gesunder Schweine und einem Datensatz von Schweinen mit Pneumothorax bestanden, d. h. jedem Pixel wurde ein Absolutwert bzw. Farbwert zugeordnet, der sich aus einer Differenz des Vergleichs entsprechend positionierter Pixel von gesunden und kranken Tieren ergibt. Auf der Ordinate ist hierbei die normierte Häufigkeit des Auftretens bestimmter Werte eines Pixels, auf der Abszisse sind die entsprechenden Werte aufgetragen. Die Datensätze wurden hinsichtlich ihrer Unterschiede verglichen, die allerdings primär bei tiefen Werten nahe Null vorliegen, während die höheren Werte im Bereich von 50-255 praktisch keine Rolle spielen. Durch die im Histogramm 13 kumulierten Unterschiede von knapp 20 Schweinepaaren kann eine Funktion gewählt werden, die den dargestellten Verlauf gut approximiert. An das Histogramm 13 kann im gezeigten Ausführungsbeispiel die Sigmoid-Funktion der Form

$$P(x) = 1 + \frac{-1}{1 + \exp(A \times (B - x))}$$

an die Daten des Histogramms 13 angefittet werden, wobei die Parameter $A$ und $B$ angepasst werden.

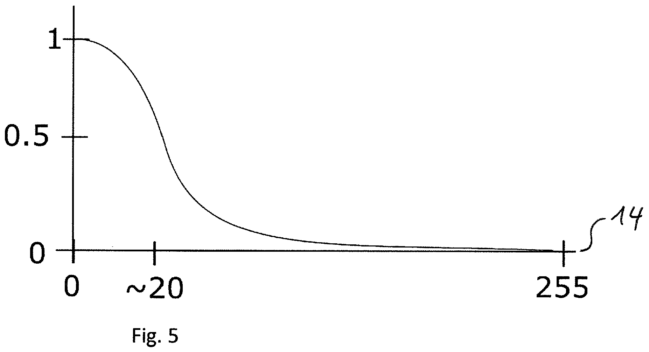

Figur 5 zeigt zur besseren Verdeutlichung ein Diagramm 14 mit einem Verlauf der angefitteten Funktion P(x) als Transformationsfunktion in Figur 4, die auch die Wahrscheinlichkeit angibt, dass der betrachtete Pixel Teil eines Pneumothorax angibt. Somit wird zwar eine Wahrscheinlichkeit angegeben, die beispielsweise einem Arzt zusätzliche Informationen liefert und auf mögliche Gefahren aufmerksam machen kann, als alleiniges Indiz für eine Diagnose im Allgemeinen jedoch nicht ausreicht. Vielmehr muss in diesem Fall ein menschlicher Benutzer die ausgegebenen Ergebnisse interpretieren und insbesondere Begleitumstände miteinbeziehen.

**Patentansprüche**

1.   Vorrichtung (3) zum Verarbeiten und Visualisieren von Daten einer elektrischen Impedanztomographie einer Lunge mit einer Aufnahmeeinheit (4), die ausgebildet ist, Daten einer elektrischen Impedanztomographie zu erfassen, oder einer Eingabeeinheit, die ausgebildet ist, mittels einer elektrischen Impedanztomographie aufgenommener Daten zur Weiterverarbeitung zu erhalten, einer Auswerteeinheit (5), die eingerichtet ist, eine visuelle Darstellung der Daten zu generieren und mindestens ein Tidalbild (12) eines Atemzyklus zu bilden, wobei jeder Pixel des mindestens einen Tidalbilds (12) anhand einer vorab definierten Funktion transformiert wird, wobei die Transformation jedem der Pixel des mindestens einen Tidalbilds (12) einen Absolutwert zuordnet, der einer Wahrscheinlichkeit für ein Auftreten einer Belüftungsstörung entspricht **dadurch gekenn-**

**zeichnet, dass** die Auswerteeinheit (5) dazu eingerichtet ist, für das Transformieren des mindestens einen Tidalbilds anhand der vorab definierten Funktion eine Sigmoid-Funktion zu verwenden.

2.   Vorrichtung nach Anspruch 1, **dadurch gekennzeichnet, dass** Parameter der Sigmoid-Funktion vorab anhand eines Histogramms (13) gewählt werden, wobei das Histogramm (13) eine Differenz zwischen Daten einer elektrischen Impedanztomographie gesunder Individuen und an dem bestimmten Ereignis erkrankter Individuen aufweist.

3.   Vorrichtung nach Anspruch 1 oder Anspruch 2, **gekennzeichnet durch** eine Ausgabeeinheit (6), die dazu eingerichtet ist, das transformierte Tidalbild (12) visuell erkennbar darzustellen, vorzugsweise in einer Falschfarbendarstellung.

4.   Vorrichtung nach einem der vorhergehenden Ansprüche, **dadurch gekennzeichnet, dass** die Auswerteeinheit (5) ausgebildet ist, unmittelbar vor dem Transformieren des mindestens einen Tidalbilds (12) anhand der vorab definierten Funktion eine Normalisierung durchzuführen, wobei als niedrigster Wert der Absolutwerte des Tidalbilds ein Mittelwert von Absolutwerten von Randpixeln des mindestens einen Tidalbilds (12) verwendet wird.

5.   Vorrichtung nach einem der vorhergehenden Ansprüche, **dadurch gekennzeichnet, dass** die Auswerteeinheit (5) eingerichtet ist, vor dem Transformieren des mindestens einen Tidalbilds (12) anhand der vorab definierten Funktion eine Filterung, vorzugsweise eine Tiefpassfilterung oder eine Bandpassfilterung zum Entfernen von Störgrößen durchzuführen.

6.   Computerprogrammprodukt, enthaltend eine Befehlsfolge, die eine Vorrichtung zum Verarbeiten und Visualisieren von Daten einer elektrischen Impedanztomographie nach einem der Ansprüche 1-5 zur Durchführung der Schritte, zu denen die Vorrichtung gemäß den Merkmalen dieser Ansprüche eingerichtet ist, ansteuert, wenn die Befehlsfolge in der Vorrichtung durchgeführt wird.

**Claims**

1.   A device (3) for processing and visualizing data of an electrical impedance tomography of a lung, comprising a recording unit (4) which is designed to record data of an electrical impedance tomography or an input unit which is designed to receive data recorded by means of an electrical impedance tomography for further processing, an evaluation unit (5) configured to generate a visual representation of the data and to form at least one tidal image (12) of a respiratory cycle, wherein each pixel of the at least one tidal image (12) is transformed using a predefined function, wherein the transformation assigns to each of the pixels of the at least one tidal image (12) an absolute value which corresponds to a probability for an occurrence of an aeration disturbance, **characterized in that** the evaluation unit (5) is configured to use a sigmoid function for transforming the at least one tidal image on the basis of the predefined function.

2. The device of claim 1, **characterized in that** parameters of the sigmoid function are selected in advance based on a histogram (13), the histogram (13) comprising a difference between data of an electrical impedance tomography of healthy individuals and individuals diseased at the particular event.

3. The device according to claim 1 or claim 2, **characterized by** an output unit (6) arranged to display the transformed tidal image (12) in a visually recognizable manner, preferably in a false color representation.

4. The device according to one of the preceding claims, **characterized in that** the evaluation unit (5) is designed to perform a normalization immediately before transforming the at least one tidal image (12) on the basis of the predefined function, wherein an average value of absolute values of edge pixels of the at least one tidal image (12) is used as the lowest value of the absolute values of the tidal image.

5. The device according to one of the preceding claims, **characterized in that** the evaluation unit (5) is configured to perform filtering, preferably low-pass filtering or band-pass filtering, for removing disturbance variables before transforming the at least one tidal image (12) on the basis of the predefined function.

6. A computer program product comprising a sequence of instructions that drives a device for processing and visualizing electrical impedance tomography data according to any one of claims 1-5 for performing the steps that the device is adapted to perform according to the features of those claims when the sequence of instructions is performed in the device.

**Revendications**

1. Dispositif (3) pour traiter et visualiser des données d'une tomographie par impédance électrique d'un poumon comprenant une unité de réception (4), qui est conçue pour recueillir des données d'une tomographie par impédance électrique, ou avec une unité d'entrée qui est conçue pour recevoir des données enregistrées au moyen d'un tomographie par impédance pour un traitement ultérieur, une unité d'évaluation (5) qui est configurée pour générer une représentation visuelle des données et pour former au moins une image de marée (12) d'un cycle respiratoire, dans laquelle chaque pixel de l'au moins une image de marée l'image (12) est transformée à l'aide d'une fonction prédéfinie, la transformation affectant à chacun des pixels de la au moins une image de marée (12) une valeur absolue qui correspond à une probabilité d'apparition d'une perturbation de ventilation, **caractérisé en ce que** l'évaluation l'unité (5) est configurée pour transformer la au moins une image de marée en utilisant la fonction précédemment définie pour utiliser une fonction sigmoïde.

2. Dispositif selon la revendication 1, **caractérisé en ce que** des paramètres de la fonction sigmoïde sont présélectionnés sur la base d'un histogramme (13), l'histogramme (13) présentant une différence entre les données d'une tomographie par impédance électrique d'individus sains et d'individus souffrant de la maladie spécifique.

3. Dispositif selon la revendication 1 ou la revendication 2, **caractérisé par** une unité de sortie (6) qui est configurée pour afficher l'image de marée transformée (12) d'une manière reconnaissable visuellement, de préférence dans un affichage en fausses couleurs.

4. Dispositif selon l'une quelconque des revendications précédentes, **caractérisé en ce que** l'unité d'évaluation (5) est conçue pour effectuer une normalisation à l'aide de la fonction définie précédemment immédiatement avant la transformation de la au moins une image de marée (12), une valeur moyenne des valeurs absolues de pixels de bord de la au moins une image de marée (12) étant utilisée comme valeur la plus basse des valeurs absolues de l'image de marée.

5. Dispositif selon l'une quelconque des revendications précédentes, **caractérisé en ce que** l'unité d'évaluation (5) est configurée pour effectuer un filtrage, de préférence un filtrage passe-bas ou un filtrage passe-bande, pour éliminer des variables d'interférence avant de transformer la au moins une image de marée (12) à l'aide de la fonction définie précédemment.

6. Produit de programme informatique, contenant une séquence d'instructions, qui commande un dispositif pour traiter et visualiser des données d'une tomographie par impédance électrique selon l'une quelconque des revendications 1 à 5 pour la réalisation des étapes pour lesquelles le dispositif est agencé selon les caractéristiques de ces revendications, lorsque la séquence d'instructions est exécutée dans le dispositif.

EP 3 967 220 B1

Fig. 2

Fig. 1

9

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0193760 A1
- DE 102015006902 B3
- DE 102016107603 A1
- EP 2762062 A1
- DE 60124541 T2

**In der Beschreibung aufgeführte Nicht-Patentliteratur**

- **GIRRBACH F ; LANDECK T ; SCHNEIDER D et al.** Detection of post-traumatic pneumothorax using electrical impedance tomography-An observer blinded study in pigs with blunt chest trauma. *PLoS One.*, 2020, vol. 15 (1), e0227518